# NOUVEAU FASCICULE DE BREVET EUROPEEN

(11) **EP 3 592 432 B2**
(45) Date de publication et mention de la décision concernant l'opposition: **17.07.2024**
(45) Mention de la délivrance du brevet: 31.03.2021
(21) Numéro de dépôt: 18711083.8
(22) Date de dépôt: 12.03.2018
(51) Int. Cl.: A61Q 5/06, A61K 8/97, A61K 8/49, A61K 8/60

(54) **EXTRAIT DE GARDÉNIA POUR LA COLORATION DES FIBRES KÉRATINIQUES**
GARDENIA EXTRAKT ZUR FÄRBUNG VON KERATINFASERN
GARDENIA EXTRACT USED FOR COLORING KERATIN FIBERS

(30) Priorité: 10.03.2017 FR 1752005
(43) Date de publication de la demande: 15.01.2020
(73) Titulaire: Pierre Fabre Dermo-Cosmétique, 81500 Lavaur (FR)
(72) Inventeur: FIORINI, Christel, 31500 Toulouse (FR); JOULIA, Philippe, 31290 Villenouvelle (FR)
(74) Mandataire: Regimbeau
(86) Numéro de dépôt international: PCT/EP2018/056094
(87) Numéro de publication internationale: WO 2018/162760

(56) Documents cités:
- EP-A1- 2 438 900
- EP-A2- 0 440 494
- WO-A1-2011/117554
- CN-A- 101 559 030
- CN-A- 106 176 453
- FR-A1- 3 003 760
- JP-A- 2013 133 320
- JP-A- H04 342 518
- KR-A- 20130 143 296
- US-A1- 2003 145 395
- US-A1- 2007 166 253
- US-A1- 2010 313 362
- "Gardenie ", WIKIPEDIA, 2 June 2020 (2020-06-02), Retrieved from the Internet <URL:https://de.wikipedia.org/w/index.php?title=Gardenie&oldid=200567846>
- XIAOYAN WUA ET AL.: "Quality control and producing areas differentiation of Gardeniae Fructus for eight bioactive constituents by HPLC-DAD-ESI/MS", PHYTOMEDICINE, vol. 21, 2014, pages 551 - 559
- Traduction de CN101559030
- Extrait CAS: CN 101559030 A {https.//scifinder-n.cas.org)
- WATANABE TOSHIRO, YAMAMOTO AKIRA, NAGAI SHLRO, TERABE SHIGERU: "Separation and Determination of Yellow Gardenia Pigments for Food and lridoid Constituents in Gardenia Fruits by Micellar Electrokinetic Chronratography", FOOD SCI. TECHNOL. INT. TOKYO, vol. 4, no. 1, 1 January 1998 (1998-01-01), pages 54 - 58
- Traduction de CN 106176453
- Extrait CAS: CN 106176453 A (https.//scifinder-n.cas.org)
- WikiPhyto.org: "Crocine’, disponible sur:[http7/www.wikiphyto.org/w/index.php?title=Crocine&oldid=23370]
- DWDS. deutscher Wortschatz, Bedeutung von "wassrig"https ://www.dwds.de/wb/wassrig
- LAN GAO AND BI-YUN ZHU: "The Accumulation of Crocin and Geniposide andTranscripts of Phytoene Synthase during Maturation ofGardenia jasminoides Fruit", EVIDENCE-BASED COMPLEMENTARY AND ALTERNATIVE MEDICINE, vol. 2013, ID686351, 2013 - 2013, pages 1 - 6, XP002771441, Retrieved from the Internet <URL:http://dx.doi.org/10.1155/2013/686351> [retrieved on 20170627]

## Description

La présente invention concerne l'utilisation d'un extrait ou d'une poudre de Gardénia destinée à colorer les fibres kératiniques, notamment les cheveux.

Parmi les méthodes de coloration des cheveux, on peut citer la coloration d'oxydation ou permanente. Ce mode de coloration met en œuvre un ou plusieurs précurseurs de colorant d'oxydation, habituellement une ou plusieurs bases d'oxydation éventuellement associées à un ou plusieurs coupleurs.

Ces bases d'oxydation sont des composés incolores ou faiblement colorés qui, associés à des produits oxydants, permettent d'accéder, par un processus de condensation oxydative, à des espèces colorées qui restent piégées à l'intérieur de la fibre capillaire.

Lors de l'utilisation d'un agent de teinture des cheveux par oxydation, le premier agent et le second agent sont mélangés immédiatement avant application et le mélange est appliqué sur les cheveux. Lors de l'application de l'agent de coloration des cheveux à l'oxydation, le colorant d'oxydation pénétré dans les cheveux est polymérisé par oxydation dans les cheveux de manière à générer un colorant indoïde volumineux et le colorant indo se développe. Le colorant indo n'est pas aisément enlevé des cheveux en raison de son encombrement, ce qui permet d'assurer une excellente rétention de la couleur des cheveux teints et d'obtenir une grande variété de teintes. Cependant, il est connu que les colorants d'oxydation peuvent provoquer des troubles cutanés. En outre, il a été souligné que les colorants d'oxydation sont des perturbateurs endocriniens qui affectent négativement un écosystème, et aussi qu'ils auraient produit des cancers, des allergies et similaires.

Les molécules colorées et colorantes peuvent également être des composés naturels issus de plantes ou d'arbres. Il est cependant difficile d'envisager toutes les nuances tinctoriales. Il existe par conséquent un réel besoin de développer des colorations capillaires à partir de produits naturels.

De façon inattendue, les inventeurs ont trouvé qu'un extrait de Gardénia, ou une poudre de Gardénia, pouvait être utile pour colorer les fibres kératiniques, notamment humaines.

Originaire de Chine et du Japon, *Gardenia jasminoides* J. Ellis est un arbuste de la famille des Rubiaceae à feuillage persistant d'environ 1 à 2 m de haut généralement. Cette plante est cultivée dans les pays tropicaux chauds et humides. Les feuilles, d'un vert foncé, sont opposées, elliptiques à ovales-oblongues, de 5 à 10 cm de longueur et de 2 à 7,5 cm de largeur, cunéiformes à la base et aiguës ou acuminées à l'apex avec un pétiole court et à stipules soudées par paires. Les fleurs blanches à ivoire, sont campanulées, de 3 à 4 cm, solitaires, terminales, sessiles et très odorantes. Le fruit est une baie coriace à 5 côtes, de 1 à 1,5 cm de long, ovoïde ou ellipsoïde, surmontée du calice persistant, jaune à rouge à maturité et contenant de nombreuses graines. Le fruit mûr, récolté en automne et séché, est inscrit à la pharmacopée chinoise.

La médecine chinoise prescrit le fruit de Gardénia sous différentes préparations : fruits secs (Zhi-zi), fruits frits (Chaozhi-zi) ou fruits carbonisés (Jiaozhi-zi). En usage interne, il est prescrit comme antipyrétique, contre la dysenterie bacillaire, les infections pulmonaires et urinaires, les hépatites ou comme hémostatique dans les hémorragies nasales provoquées par la fièvre et en usage externe pour traiter les blessures, l'inflammation oculaire, les contusions, les plaies et les furoncles. Dans la médecine japonaise Kampo le fruit est utilisé pour traiter la douleur, les affections pulmonaires et la jaunisse. Ces usages traditionnels peuvent être expliqués par les propriétés pharmacologiques du fruit qui est hémostatique, anti-inflammatoire, stimulant, cholagogue, émétique et diurétique.

Les autres parties du *Gardenia jasminoides* présentent de multiples vertus. Les feuilles, fébrifuges, sont écrasées en Malaisie pour confectionner des cataplasmes pour traiter les migraines, les inflammations pulmonaires. L'écorce, fébrifuge et tonique, est utilisée en cas de fièvre, dysenterie et de douleurs abdominales. En Inde, la racine est utilisée dans la dyspepsie et dans les désordres nerveux. Les fleurs, émollientes, sont utiles pour traiter l'ophtalmie, la blennorragie et la vaginite. Les graines sont utilisées en externe sous forme de pâte pour traiter la jaunisse, le rhumatisme, la diverticulose.

En complément de ces usages médicinaux, les fruits sont également employés pour colorer les denrées alimentaires ou les textiles de couleur jaune à orange en raison de leur richesse en crocines, des pigments identiques à ceux du safran.

Les principaux composés du fruit sont :
➢ des iridoïdes, représentés majoritairement par le géniposide et gardénoside. Les autres iridoïdes présents ainsi que les composés suivants en quantité plus faible: 6"-O-*trans*-sinapoylgénipin gentiobioside, 6"-O-*trans*-p-coumaroylgenipin gentiobioside, 6"-O-*trans*-cinnamoylgénipin gentiobioside, 6"-O-*trans*-p-coumaroylgéniposide, acide 6'-O-*trans*-p-coumaroylgéniposide, 10-O-succinoylgéniposide, 6'-O-acetylgéniposide, Gardenal-I, Gardenal-II, Gardenal-III, 6-β-hydroxygéniposide,6-α-hydroxygéniposide,6-α-méthoxygéniposide, férétoside, génipin-1-β-gentiobioside, shanzhiside et les acides lamalbidique et picrocrocinique.
➢ des caroténoïdes tels que l'acide crocéique, la crocétine et les crocines, dérivés glycosylés de la crocétine. On distingue la crocine 1 (crocétine gentiobioside), la crocine 2 (crocétine gentiobioside glucoside) et la crocine 3 (crocétine glucoside),
➢ des flavonoïdes : gardénine, quercétine, quercétine-3-rutinoside, quercétine-3-O-glucopyranoside, isoquercitrine, corymbosine, umuhengérine, nicotiflorine.
➢ des dérivés caféylquiniques (acide 3-caféoylquinique, acide 4-caféoylquinique), acide 3,4-dicaféoylquinique, acide 3,5-dicaféoylquinique, acide 4,5-dicaféoylquinique, 5-*O*-caféoylquinate d'éthyle, 5-*O*-caféoyl 3-*O*-sinapoylquinate de méthyl, 5-*O*-caféoyl 3-*O*-sinapoylquinate d'éthyle, 5-*O*-caféoyl 4-*O-*sinapoylquinate de méthyl, 5-*O*-caféoyl 4-*O*-sinapoylquinate d'éthyle, 3,5-di-*O*-caféoyl-4-*O*-(3-hydroxy-3-méthyl)glutaroylquinate de méthyle, acide 3-O-caféoyl-4-O-sinapoylquinique,3-O-caféoyl-4-O-sinapoylquinate de méthyle, 3-O-caféoyl-5-O-sinapoylquinate de méthyle, acide 3,4-di-O caféoyl-5-O-(3-hydroxy-méthyl) glutaroylquinique, acide 3,5-di-O-caféoyl-4-O-(3-hydroxyméthyl) glutaroylquinique.
➢ des acides phénols tels que les acides chlorogénique, caféique et 3,4-dihydroxy-benzoique.
➢ des lignanes : gardénianane A, syringarésinol, pinorésinol, syringarésinol-4-O-β-D-glucopyranoside, laricirésinol, alangilignoside D, lyonirésinol, lyonirésinol-9-O-β-D-glucopyranoside, balanophonine, acide glycosmisique, ficusal et céplignane.
➢ des sucres (mannitol).

La présente invention concerne l'utilisation d'un extrait ou poudre de Gardénia pour colorer les fibres kératiniques, notamment humaines.

Selon un mode de réalisation, l'invention vise ainsi l'utilisation d'un extrait de Gardénia, en particulier un extrait de fruits de Gardénia pour colorer les fibres kératiniques, notamment les fibres kératiniques humaines, plus particulièrement encore les cheveux. L'invention vise plus particulièrement l'utilisation d'un extrait de Gardénia pour la coloration des cheveux en blond. Plus particulièrement l'invention vise la coloration en blond de cheveux préalablement décolorés.

Selon un mode de réalisation, l'invention vise ainsi l'utilisation d'une poudre de Gardénia, en particulier une poudre de fruits de Gardénia, lesdits fruits étant de préférence préalablement séchés, pour colorer les fibres kératiniques, notamment les fibres kératiniques humaines, plus particulièrement encore les cheveux. L'invention vise plus particulièrement l'utilisation d'une poudre de Gardénia, en particulier une poudre de fruits de Gardénia, en particulier de fruits secs, pour la coloration des cheveux en blond. Plus particulièrement l'invention vise la coloration en blond de cheveux préalablement décolorés.

L'invention porte sur l'utilisation d'une composition cosmétique pour colorer les cheveux en blond selon la revendication 1.

### Mode de réalisation décrits

### Modes de réalisation de l'invention

1. Utilisation d'un extrait de Gardénia pour colorer les fibres kératiniques, notamment les fibres kératiniques humaines, plus particulièrement encore les cheveux, caractérisé en ce que l'extrait contient entre 0,1 et 10% de crocines en poids, préférentiellement entre 1 et 5% en poids, par rapport au poids de l'extrait sec.
2. Utilisation selon le mode de réalisation 1, caractérisé en ce que l'extrait est un extrait de fruits de Gardenia.
3. Utilisation selon l'un des modes de réalisation 1 à 2, caractérisée en ce que l'extrait est un extrait aqueux ou alcoolique ou hydroalcoolique.
4. Utilisation selon l'un des modes de réalisation 1 à 3, caractérisée en ce que l'extrait est un extrait sec.
5. Utilisation selon l'un des modes de réalisation 1 à 4, pour colorer les cheveux en blond, plus particulièrement pour colorer en blond les cheveux préalablement décolorés.
6. Utilisation d'une composition cosmétique destinée à colorer les fibres kératiniques, notamment humaines, ladite composition cosmétique comprenant à titre de principe actif un extrait de Gardénia tel que défini dans l'un des modes de réalisation 1 à 4.
7. Utilisation selon le mode de réalisation 6, caractérisée en ce que la composition comprend outre l'extrait de Gardénia comme principe actif colorant, un excipient cosmétiquement acceptable.
8. Utilisation selon l'un des modes de réalisation 6 ou 7, caractérisée en ce que la composition présente un pH compris entre 4 et 8.
9. Utilisation selon l'un des modes de réalisation 6 à 8, caractérisée en ce que la composition est dénuée de composé réducteur.
10. Utilisation selon l'un des modes de réalisation 6 à 9, caractérisée en ce que la composition est dénuée de composé alcalin fixateur.
11. Utilisation d'une poudre de Gardénia, pour colorer les fibres kératiniques, notamment les fibres kératiniques humaines, plus particulièrement encore les cheveux caractérisée en ce que la poudre de Gardénia contient, entre 0,1 et 10% en poids de crocines, préférentiellement entre 1 et 5% en poids, par rapport au poids de la poudre.
12. Utilisation selon le mode de réalisation 11, caractérisé en ce que la poudre est une poudre de fruits de Gardénia, de préférence de fruits séchés de Gardénia.
13. Utilisation selon l'un des modes de réalisation 11 à 12, pour colorer les cheveux en blond, plus particulièrement pour colorer en blond les cheveux préalablement décolorés.
14. Utilisation d'une composition cosmétique destinée à colorer les fibres kératiniques, notamment humaines, ladite composition cosmétique comprenant à titre de principe actif une poudre de Gardénia tel que définie dans l'un des modes de réalisation 11 à 12.
15. Utilisation selon le mode de réalisation 14, caractérisée en ce que la composition comprend outre la poudre de Gardénia comme principe actif colorant, un excipient cosmétiquement acceptable.
16. Utilisation selon l'un des modes de réalisation 14 ou 15, caractérisée en ce que la composition présente un pH compris entre 4 et 8.
17. Utilisation selon l'un des modes de réalisation 14 à 16, caractérisée en ce que la composition est dénuée de composé réducteur.
18. Utilisation selon l'un des modes de réalisation 14 à 17, caractérisée en ce que la composition est dénuée de composé alcalin fixateur.
19. Kit comprenant un extrait de Gardénia tel que défini dans un des modes de réalisation 1 à 4, un excipient cosmétiquement acceptable et une notice d'emploi pour une utilisation pour la coloration de cheveux, en particulier la coloration des cheveux en blond.
20. Kit comprenant une poudre de Gardénia telle que définie dans un des modes de réalisation 11 à 12, un excipient cosmétiquement acceptable et une notice d'emploi pour une utilisation pour la coloration de cheveux, en particulier la coloration des cheveux en blond.
21. Méthode de traitement cosmétique destinée à colorer les fibres kératiniques, notamment les cheveux humains, en particulier en blond, consistant en l'administration d'une composition cosmétique telle que définie dans l'un des modes de réalisation 6 à 10.
22. Méthode de traitement cosmétique destinée à colorer les fibres kératiniques, notamment les cheveux humains, en particulier en blond, consistant en l'administration d'une composition cosmétique telle que définie dans un des modes de réalisation 14 à 18.
24. Méthode de coloration des fibres kératiniques, en particulier les cheveux, et plus particulièrement la coloration des cheveux en blond, comprenant les étapes :
   a) application d'une composition cosmétique comprenant un extrait de Gardénia tel que défini dans l'un des modes de réalisation 1 à 4 ou une poudre de Gardénia telle que définie dans l'un des modes de réalisation 11 à 12,
   b) temps de pause compris entre 15 min et 3 heures,
   c) rinçage à l'eau,
   d) éventuelle répétition des étapes a) à c),
   e) éventuel séchage.
23. Méthode de coloration selon le mode de réalisation 24, caractérisée en ce qu'elle comprend une étape optionnelle, avant l'étape a) consistant à la préparation extemporanée de la composition par mélange des composants d'un kit tel que défini dans l'un des modes de réalisation 19 ou 20 ou par mélange d'un extrait de Gardénia tel que défini dans l'un des modes de réalisation 1 à 4 avec de l'eau ou encore par mélange d'une poudre de Gardénia telle que définie dans l'un des modes de réalisation 11 à 12 avec de l'eau.
24. Utilisation d'un extrait de Gardénia selon l'un des modes de réalisation 1 à 5 ou utilisation d'une composition selon l'un des modes de réalisation 6 à 10, caractérisé en ce que l'extrait est obtenu par un procédé de préparation comprenant les étapes suivantes :
   - une extraction à partir de fruits de Gardénia, en particulier de fruits séchés, optionnellement en présence de pectinases, par un solvant choisi dans le groupe constitué par l'eau, l'éthanol et l'acétone, ainsi que leurs mélanges,
   - une séparation solide-liquide,
   - une éventuelle stérilisation du filtrat, et
   - éventuellement évaporation du solvant à des températures inférieures à 80 °C.
25. Utilisation selon le mode de réalisation 24, caractérisé en ce que le solvant est choisi dans le groupe constitué par l'eau, l'éthanol et leurs mélanges.
26. Utilisation selon l'un des modes de réalisation 24 ou 25, caractérisé en ce que l'étape d'extraction est réalisée à un pH compris entre 4 et 8, de préférence, entre 5 et 7,5, avantageusement entre 5,5 et 7,5, typiquement à pH neutre.

Par coloration en blond, on entend au sens de la présente invention, une coloration en blond (N°7), en blond clair (N°8), blond très clair (N°9) ou blond platine (N°10) tel que défini tels qu'utilisés selon l'échelle universelle (de N°1 à N° 10) pour nommer les hauteurs de ton dans la coiffure professionnelle.

La présente invention s'inscrit dans une volonté de mettre en place des voies de synthèse qui soient plus vertes et qui permettent de revendiquer la naturalité des principes actifs ainsi obtenus. Ainsi, le ou les solvants utilisés dans le cadre de la présente invention seront donc de préférence des solvants naturels et/ou d'origine naturelle issus de ressources renouvelables, par opposition aux ressources fossiles, ces solvants pouvant de manière avantageuse être obtenus par des procédés respectueux de l'environnement. L'extrait ainsi obtenu selon le procédé de l'invention sera donc de préférence un extrait naturel et/ou d'origine naturelle, issu de ressources renouvelables, par opposition aux ressources fossiles.

Dans un mode de réalisation préféré, le procédé mis en œuvre permet de conserver les molécules d'intérêt, les crocines, sous leur forme native, c'est-à-dire glycosylée. La coloration est donc obtenue grâce aux pigments naturellement présents dans la plante. Ainsi le procédé selon l'invention ne comporte aucune étape consistant à ajouter de la b-glucosidase et/ou un acide aminé.

Un extrait de Gardenia selon l'invention peut en outre contenir tout composé naturellement présent dans les fruits de *Gardenia.* Dans un mode de réalisation particulier, un extrait selon l'invention ne contient pas plus de 10 %, en pourcentage en poids, de géniposide par rapport au poids total de l'extrait sec, en particulier entre 7 et 10% en poids. Lorsque l'on parle de poids total de l'extrait sec, il s'agit d'extrait sec de Gardénia, hors éventuel support neutre de type maltodextrine par exemple.

L'extrait de Gardénia selon l'invention peut être un extrait aqueux ou alcoolique ou hydroalcoolique.

La présente invention concerne l'utilisation d'une composition cosmétique comprenant à titre de principe actif un extrait de Gardénia, extrait tel que décrit ci-avant.

L'utilisation de la composition cosmétique vise la coloration des cheveux en blond. Plus particulièrement l'invention vise la coloration en blond de cheveux préalablement décolorés.

L'extrait de Gardénia selon l'invention peut se présenter sous une forme liquide ou fluide, c'est-à-dire que tout ou partie du solvant d'extraction est toujours présent.

L'extrait de Gardénia selon l'invention peut se présenter sous la forme d'un extrait sec, c'est-à-dire que le solvant d'extraction a été oté, par exemple évaporé, pour obtenir un extrait sec. L'Extrait de Gardénia selon l'invention est miscible ou soluble dans l'eau, selon que l'on parle d'un extrait liquide ou d'un extrait sec, respectivement. Selon la quantité d'eau ajoutée l'extrait obtenu peut ainsi se présenter sous la forme d'une solution liquide, fluide ou plus ou moins visqueuse.

L'extrait de Gardenia selon l'invention peut-être un extrait fluide ou pâteux, aqueux ou hydro-alcoolique. Il peut s'agir d'un extrait sec, aqueux ou hydro-alcoolique. En particulier il s'agit d'un extrait aqueux.

Dans un autre mode de réalisation, l'invention vise l'utilisation d'une composition cosmétique destinée à colorer les fibres kératiniques, notamment humaines, ladite composition cosmétique comprenant à titre de principe actif un extrait fluide de Gardenia.

Dans un autre mode de réalisation, l'invention vise l'utilisation d'une composition cosmétique destinée à colorer les fibres kératiniques, notamment humaines, ladite composition cosmétique comprenant à titre de principe actif un extrait sec de Gardenia.

Selon un mode particulier de l'invention, un extrait fluide ou liquide de Gardenia, plus particulièrement un extrait aqueux ou hydro-alcoolique de *Gardénia,* selon l'invention peut ainsi se présenter sous la forme de la fraction liquide ou fluide (plus ou moins visqueuse) obtenue après extraction et séparation liquide-solide et contenant de 20 à 60% en poids de matière sèche de Gardénia, représentant donc l'extrait sec, et plus particulièrement contenant de 30 à 50% en poids de matière sèche de Gardénia, représentant donc l'extrait sec et plus particulièrement encore contenant environ 40% en poids de matière sèche de Gardénia, représentant donc l'extrait sec dans le solvant aqueux ou hydro-alcoolique. L'extraction est réalisée sur des fruits de Gardénia par des techniques connues et détaillées ci-après.

Selon un autre mode particulier de l'invention, l'extrait selon l'invention peut aussi se présenter sous la forme d'extrait sec une fois le solvant, aqueux ou hydro-alcoolique, éliminé, par exemple par évaporation, de l'extrait fluide ou liquide. Cet extrait sec est typiquement pulvérulent présentant une granulométrie moyenne comprise entre 0.1 µm et 250 µm, particulièrement entre 1 µm et 250 µm Par « extrait sec », on entend, au sens de la présente invention, un extrait dépourvu de solvant d'extraction, ou en contenant uniquement à l'état de trace non significative. Un tel extrait sec contient ainsi uniquement de la matière issue de *Gardenia*, en particulier de fruits de *Gardénia.*

La composition cosmétique selon l'invention comprend ainsi, outre l'extrait de Gardénia tel que défini plus avant comme principe actif colorant, un excipient cosmétiquement acceptable.

La composition cosmétique selon l'invention présente un pH compris entre 4 et 8, de préférence, entre 5 et 7,5, avantageusement entre 5,5 et 7,5, typiquement un pH neutre.

Par « pH neutre », on entend un pH compris entre 6.5 et 7.5, particulièrement aux environs de 7.

La composition selon l'invention est dénuée de composé réducteur de type thiol par exemple.

La composition selon l'invention est en particulier dénuée de composé alcalin fixateur de type ammoniac par exemple.

C'est en effet un grand avantage et une caractéristique particulièrement avantageuse des compositions selon l'invention que d'être fonctionnelles, c'est-à-dire permettre une coloration puissante et durable dans le temps tout en étant dénuée des additifs de type agent réducteur ou fixateur alcalin couramment rencontrés dans les formulations colorantes de l'art antérieur. Il est connu que ces additifs sont susceptibles d'affecter l'intégrité de la fibre sur le long terme et d'irriter le cuir chevelu. Les extraits et compositions selon l'invention permettent ainsi une coloration rapide, efficace et durable sans encourir les problèmes associés à la présence de ces composés fixateurs ou réducteurs. La méthode de coloration ainsi proposée selon l'invention permet de colorer la fibre capillaire tout en conservant son intégrité et son état naturel.

Par excipient cosmétiquement acceptable on entend au sens de la présente invention un excipient contenant des ingrédients adaptés pour la formulation de compositions cosmétiques du type crème, lotion, shampooing, émulsion ou toute formulation adaptée pour une application sur les cheveux et le cuir chevelu.

L'invention vise aussi l'utilisation d'une poudre de Gardénia, plus particulièrement une poudre de fruit de Gardénia, en particulier de fruits secs, pour la coloration de cheveux, en particulier pour colorer les cheveux en blond. Plus particulièrement l'invention vise la coloration en blond de cheveux préalablement décolorés.

Dans un mode de réalisation particulier, on entend par « poudre de Gardénia » un produit naturel pur provenant des fruits de Gardenia, en particulier fruits secs, réduits, par broyage ou autres moyens mécaniques, en fines particules de granulométrie moyenne comprise entre 0.1 µm et 250 µm, particulièrement entre 1 µm et 250 µm. Le broyage desdits fruits de Gardénia pour obtenir une poudre peut avantageusement être réalisé par broyage des fruits secs par tout moyen adapté permettant une réduction de taille et l'obtention de fines particules telles qu'évoquée ci-avant.

Dans un mode de réalisation préféré, une poudre selon l'invention est un produit sec et pulvérulent dont le taux d'humidité est négligeable.

Une poudre selon l'invention est ainsi hydrosoluble, c'est-à-dire qu'elle peut servir pour l'obtention d'une composition liquide aqueuse ou hydroalcoolique contenant 20 à 60% en poids de matière sèche, plus particulièrement entre 30 et 50% en poids de matière sèche et plus particulièrement encore environ 40% en poids de matière sèche. La matière sèche représente ainsi la poudre de Gardénia.

Une poudre selon l'invention peut en outre contenir tout composé naturellement présent dans les fruits de *Gardenia*.

Dans un mode de réalisation particulier, une poudre de Gardénia selon l'invention ne contient pas plus de 10 %, en pourcentage en poids, de géniposide, par rapport au poids total de la poudre de Gardénia, en particulier entre 1 et 5% en poids.

Par fibres kératiniques on entend les cheveux, les poils, les cils, les sourcils plus particulièrement les cheveux.

Dans un autre mode de réalisation, l'invention vise l'utilisation d'une composition cosmétique comprenant à titre de principe actif une poudre de Gardenia telle que définie ci-avant L'invention vise la coloration en blond de cheveux, les cheveux pouvant être préalablement décolorés.

En particulier, l'invention vise l'utilisation d'une composition cosmétique comprenant à titre de principe actif une poudre de Gardenia. L'utilisation selon l'invention vise la coloration des cheveux en blond. Plus particulièrement l'invention vise la coloration en blond de cheveux préalablement décolorés.

La composition cosmétique selon l'invention comprend ainsi, outre la poudre de Gardénia telle que définie plus avant comme principe actif colorant, un excipient cosmétiquement acceptable.

La composition selon l'invention est en particulier dénuée de composé réducteur de type thiol par exemple.

La composition selon l'invention est en particulier dénuée de composé alcalin fixateur de type ammoniac par exemple.

C'est en effet un grand avantage et une caractéristique particulièrement avantageuse des compositions selon l'invention que d'être fonctionnelles, c'est-à-dire permettre une coloration puissante et durable dans le temps tout en étant dénué des additifs de type agent réducteur ou fixateur alcalin couramment rencontrés dans les formulations colorantes de l'art antérieur. Il est connu que ces additifs sont susceptibles d'affecter l'intégrité de la fibre sur le long terme et d'irriter le cuir chevelu. Les poudres et compositions selon l'invention permettent ainsi une coloration rapide, efficace et durable sans encourir les problèmes associés à la présence de ces composés fixateurs ou réducteurs. La méthode de coloration ainsi proposée selon l'invention permet de colorer la fibre capillaire tout en conservant son intégrité et son état naturel.

D'une façon préférée, l'extrait de Gardenia provient de l'espèce *Gardenia jasminoides* ; et plus préférentiellement la variété radicans de l'espèce *Gardenia jasminoides.* C'est une variété particulièrement riche en crocines.

Aussi, de manière préférée, la poudre de Gardenia peut provenir de Gardenia jasminoides ; et plus préférentiellement la variété radicans de l'espèce *Gardenia jasminoides*. C'est une variété particulièrement riche en crocines.

L'extrait ou la poudre de Gardénia utilisé pour colorer les les cheveux, contient en pourcentage en poids, entre 1 et 10% de crocines, préférentiellement entre 1 et 5%, par rapport au poids de l'extrait sec ou de la poudre.

L'extrait ou la poudre de Gardenia selon l'invention, est utilisé pour colorer les cheveux, en blond. Plus particulièrement l'invention vise la coloration en blond de cheveux préalablement décolorés.

La composition selon l'invention peut être formulée pour l'administration à l'être humain. Les compositions selon l'invention peuvent être administrées par voie topique au niveau des fibres kératiniques. Avantageusement, la composition selon la présente invention est destinée à une administration par voie topique.

Selon un mode particulièrement intéressant de l'invention, la composition cosmétique comprend au moins un excipient cosmétiquement acceptable, en particulier un excipient viscosifiant.

Dans la présente invention, on entend désigner par « cosmétiquement acceptable » ce qui est utile dans la préparation d'une composition cosmétique, qui est généralement sûr, non toxique et ni biologiquement ni autrement non souhaitable et qui est acceptable pour une utilisation cosmétique, notamment par application topique sur les cheveux et le cuir chevelu.

Les compositions selon l'invention sont avantageusement destinées à une application topique, en particulier sur les cheveux et le cuir chevelu.

Dans ce dernier cas l'extrait de Gardenia peut être administré sous formes unitaires d'administration, en mélange avec de l'eau ou des supports cosmétiques classiques, en particulier viscosifiants, adaptées aux êtres humains. Les formes unitaires d'administration appropriées comprennent les formes par voie topique.

Dans le cas où l'on utilise de la poudre de Gardénia, celle-ci peut être administrée sous formes unitaires d'administration, en mélange avec de l'eau ou des supports cosmétiques classiques, en particulier viscosifiants adaptées aux êtres humains. Les formes unitaires d'administration appropriées comprennent les formes par voie topique.

Ainsi, selon un mode de réalisation, l'invention concerne un kit comprenant un extrait de Gardénia selon l'invention, un excipient cosmétiquement acceptable et une notice d'emploi pour une utilisation pour la coloration de cheveux, en particulier la coloration des cheveux en blond. Dans un cas particulier, l'excipient cosmétiquement acceptable peut être de l'eau.

Ainsi, selon un mode de réalisation, l'invention concerne un kit comprenant une poudre de Gardénia selon l'invention, un excipient cosmétiquement acceptable et une notice d'emploi pour son utilisation pour la coloration des cheveux en blond. Dans un cas particulier, l'excipient cosmétiquement acceptable peut être de l'eau.

Dans un mode de réalisation particulier de l'invention, la composition comprend, par dose unitaire, de 10 mg à 100 g d'extrait sec de Gardenia ou de poudre de Gardénia, préférentiellement de 20 mg à 100 g, avantageusement de 50 mg à 100 g et plus préférentiellement de 100 mg à 100 g, encore plus particulièrement de 200 mg à 75 g.

Dans un mode particulier de l'invention, la composition comprend, par dose unitaire, de 0,2 mg à 5 g de crocines, préférentiellement de 1 mg à 5 g et plus préférentiellement de 2 mg à 2 g.

L'extrait ou poudre de Gardenia contient, en pourcentage en poids par rapport au poids sec de l'extrait (hors support de séchage) ou de la poudre, entre 1 et 10% de crocines, préférentiellement entre 1 et 8%, de manière préférée entre 1 et 5%, encore plus préférentiellement entre 1 et 3%.

Selon un mode particulièrement intéressant de l'invention, la composition cosmétique comprend en outre au moins un autre agent de coloration, en particulier issu de plantes, de microorganismes ou de micro-algues.

La présente invention concerne également une méthode de traitement cosmétique destinée à colorer les fibres kératiniques, en blond, consistant en l'administration d'une composition cosmétique comprenant un extrait de Gardenia selon l'invention. De manière préférée, l'administration consiste en une application topique au niveau des cheveux et du cuir chevelu. Plus particulièrement l'invention vise la coloration en blond de cheveux préalablement décolorés.

La présente invention concerne encore une méthode de traitement cosmétique destinée à colorer les fibres kératiniques, en blond, consistant en l'administration d'une composition cosmétique comprenant de la poudre de Gardénia selon l'invention. De manière préférée, l'administration consiste en une application topique au niveau des cheveux et du cuir chevelu. Plus particulièrement l'invention vise la coloration en blond de cheveux préalablement décolorés.

L'invention vise encore une méthode de coloration des des cheveux en blond, comprenant les étapes :
a). application d'une composition cosmétique comprenant un extrait ou poudre de Gardénia selon l'invention,
b). temps de pause compris entre 15 min et 3 heures,
c). rinçage à l'eau,
d). éventuelle répétition des étapes a) à c),
e). éventuel séchage.

Selon un mode de réalisation, la méthode de coloration des cheveux en blond, peut comprendre une étape optionnelle, avant l'étape a) consistant à la préparation extemporanée d'une composition par mélange des composants d'un kit qui comprend un extrait de Gardénia selon l'invention et un excipient cosmétiquement acceptable pour son utilisation pour la coloration de cheveux.

Enfin, la méthode de coloration des cheveux en blond, peut comprendre une étape optionnelle, avant l'étape a) consistant à la préparation extemporanée d'une composition par mélange des composants d'un kit qui comprend une poudre de Gardénia selon l'invention et un excipient cosmétiquement acceptable pour son utilisation pour la coloration de cheveux.

On peut tout aussi bien préparer une composition par mélange de la poudre avec de l'eau ou par mélange de l'extrait avec de l'eau.

L'étape de pause b) peut avantageusement être réalisée à une température comprise entre 30°C et 100°C (par exemple à l'aide d'un casque séchant), plus particulièrement entre 30°C et 75°C, plus particulièrement encore aux environs de 40°C à 60°C, et encore plus particulièrement aux environs de 50°C.

Plus particulièrement l'invention vise une méthode pour la coloration en blond de cheveux préalablement décolorés.

La méthode de coloration capillaire selon l'invention peut comprendre une application répétée de la composition cosmétique selon l'invention.

La méthode de coloration capillaire pourra comprendre une étape de rinçage entre les applications répétées de la composition cosmétique selon l'invention.

Dans un mode réalisation particulier, le procédé de coloration selon l'invention ne comporte aucune étape consistant à faire varier le pH de la composition par ajout d'acide ou de base avant application sur les cheveux. En particulier, le procédé de coloration est réalisé à un pH compris entre 4 et 8, de préférence, entre 5 et 7,5, avantageusement entre 5,5 et 7,5, typiquement à pH neutre.

Par « pH neutre », on entend un pH compris entre 6.5 et 7.5, particulièrement aux environs de 7.

De même, il n'est pas nécessaire de mettre un composé fixateur (alcalin type ammoniac), ou composé réducteur (par exemple thiol).

La présente invention concerne enfin un procédé de préparation d'extrait de Gardénia, particulièrement des fruits de Gardénia.

Dans un mode de réalisation de l'invention l'extrait de Gardénia est obtenu par :
- une extraction avec solvant aqueux, organique ou hydroalcoolique à partir des fruits entiers, optionnellement en présence de pectinases,
- une séparation solide-liquide,
- une éventuelle stérilisation du filtrat, et
- éventuellement évaporation du solvant à des températures inférieures à 80 °C.

Les fruits de Gardénia sont extraits par un solvant choisi dans le groupe constitué par l'eau, un solvant organique tel que l'éthanol, ou l'acétone, ainsi que leurs mélanges. L'extraction peut être réalisée sur des fruits entiers ou broyés grossièrement au préalable. L'ajout d'enzymes, telles que des pectinases permettant d'améliorer l'extraction et/ou leur filtration en fluidifiant les jus d'extraction. Le procédé est particulièrement caractérisé par l'absence de glycosidase afin d'éviter la formation de crocétine. L'extraction peut être réalisée par une méthode classique connue de l'homme du métier, en réacteur, par ultrasons ou encore par microondes à une température comprise entre 20°C et 100°C suivant la présence ou non d'enzymes. L'extraction peut être menée à pression atmosphérique ou sous pression avec de l'eau sub-critique.

Après séparation solide-liquide, la fraction liquide (ie le filtrat) peut être concentrée stérilisée et peut être séchée en l'état ou sur un support de séchage, comme la maltodextrine ou la silice. Le séchage est réalisé à des températures inférieures à 80°C afin d'éviter la dégradation des crocines, molécules instables à des températures plus élevées. Il peut être réalisé par des techniques connues de l'homme du métier comme par exemple par micro-ondes, lyophilisation ou atomisation.

Selon un mode de réalisation préféré, le procédé selon l'invention ne comporte aucune étape consistant à faire varier le pH de la solution aqueuse par ajout d'acide ou de base.

En particulier, l'étape d'extraction est réalisée à un pH compris entre 4 et 8, de préférence, entre 5 et 7,5, avantageusement entre 5,5 et 7,5, typiquement à pH neutre.

Par « pH neutre », on entend un pH compris entre 6.5 et 7.5, particulièrement aux environs de 7.

Les exemples suivants illustrent l'invention sans en limiter la portée.

### Exemple 1 : Préparation de l'extrait de Gardénia

Les fruits entiers de Gardénia sont extraits à 90°C par l'eau pendant 2 heures. Après séparation solide/liquide, des pectinases sont additionnées. Le filtrat est concentré sur eau *q.s.p.* 40% d'extrait sec, stérilisé puis séché aux micro-ondes.

L'extrait sec obtenu est une poudre de couleur rouge. Le rendement est de 25% environ. Les teneurs en crocines sont comprises entre 0,1 et 10% en poids de l'extrait sec, la teneur moyenne en crocines est de l'ordre de 2%.

Les fruits de Gardénia sont extraits par de l'eau préférentiellement ou un mélange d'eau et d'un solvant organique, un alcool tel que l'éthanol ou une cétone telle que l'acétone. L'extraction peut être réalisée sur des fruits entiers ou broyés grossièrement au préalable. L'ajout d'enzymes, telles que des pectinases permettant d'améliorer la filtration peut être réalisée. L'extraction peut être réalisée par une méthode classique connue de l'homme du métier, en réacteur, par ultrasons ou encore par l'eau subcritique ou par microondes à une température comprise entre 20°C et 100°C suivant la présence ou non d'enzyme. Après séparation solide-liquide, le filtrat est utilisé tel quel ou concentré de préférence jusqu'à 2 volumes d'eau pour 1 partie de plante engagée, et est de préférence stérilisé. On peut ensuite sécher en l'état (ie sans support de séchage) par les méthodes classiques (sécheur à palette, lyophilisation ou micro-ondes ou atomisation) ou sur un support de séchage, comme la maltodextrine ou la silice. L'utilisation de la maltodextrine permet d'obtenir une meilleure maniabilité. L'extrait aqueux sec ainsi obtenu est une poudre de couleur rouge. Le rendement est de 25% environ. La réalisation d'1 kg d'extrait nécessite l'emploi de 4 kg de fruits de Gardénia. Les teneurs en crocines sont comprises entre 0,1 à 10% en poids par rapport à l'extrait sec, la teneur moyenne en crocines est de l'ordre de 2%. Ces molécules étant sensibles à la chaleur, la température de séchage est préférentiellement inférieure à 80°C.

### Exemple 2 : Fruits de Gardénia et/ou Poudre de fruit de Gardénia

Les fruits de Gardénia séchés sont utilisés en l'état ou broyés avec une granulométrie inférieure à 250 µm préférentiellement.

### Exemple 3 : Mise en évidence de l'efficacité colorante sur cheveux humains décolorés.

### Composition colorante 1 :

L'extrait de Gardénia selon l'exemple 1 est mélangé avec de l'eau (ratio massique 1/20 à 1/10 préférentiellement) à 50°C ou un agent viscosant tel des mucilages naturels (gomme xanthane, Ispaghul, Lin, Gombo, Konjac, Hibiscus, Calendula, Banane, Baobab, Acanthe, Aloes...).

### Procédé de coloration sur mèche de cheveu préalablement décolorée (mèche de référence):

La mèche est entièrement trempée dans cette solution visqueuse puis mise à 50 °C pendant 30 min. La mèche est ensuite rincée à l'eau chaude.

### Composition colorante 2 :

La poudre de fruit de Gardénia broyée avec une granulométrie inférieure à 250 µm préférentiellement est mélangée avec de l'eau (ratio massique 1/20 à 1/10 préférentiellement) à 50°C ou un agent viscosant tel des mucilages naturels (gomme xanthane, Ispaghul, Lin, Gombo, Konjac, Hibiscus, Calendula, Banane, Baobab, Acanthe, Aloes...).

### Procédé de coloration sur mèche de cheveu préalablement décolorée (mèche de référence):

La mèche est entièrement trempée dans cette solution visqueuse puis mise à 50 °C pendant 30 min. La mèche est ensuite rincée à l'eau chaude.

### Résultats :

Les mèches traitées sont colorées en blond dont l'intensité (de blond clair à blond platine) et la profondeur varient selon la durée et le nombre d'applications comparativement à la mèche de référence.

Les exemples réalisés ci-avant avec des mèches de cheveux préalablement décolorées ont été reproduits avec des mèches vierges, non préalablement décolorées et les résultats obtenus sont en tous points comparables en ce qui concerne la qualité et la persistance de la coloration.

### Contre-exemple : géniposide

Préparation d'une composition comprenant 10 mg de géniposide (fournisseur Sigma Aldrich) dilué dans 1 g de gomme xanthane à 2%.

### Procédé de coloration sur mèche de cheveu préalablement décolorée (mèche de référence):

La mèche est entièrement trempée dans cette solution visqueuse puis mise à 50 °C pendant 30 min. La mèche est ensuite rincée à l'eau chaude.

Résultat : nous n'observons pas d'effet colorant comparativement à la mèche de référence.

### Contre-exemple : crocétine

La crocétine est obtenue à partir de l'extrait aqueux de Gardenia, lequel a été hydrolysé par hydrolyse acide puis purifié par chromatographie.

Préparation d'une composition comprenant 10 mg de crocétine dilué dans 1 g de gommexanthane à 2%.

### Procédé de coloration sur mèche de cheveu préalablement décolorée (mèche de référence):

La mèche est entièrement trempée dans cette solution visqueuse puis mise à 50 °C pendant 30 min. La mèche est ensuite rincée à l'eau chaude.

Résultat : nous n'observons pas d'effet colorant comparativement à la mèche de référence.

### Contre-exemple : Gardenia Blue

Préparation d'une composition comprenant 0,5g de Gardenia Blue (E600764 Gardénia Blue Fournisseur JIANGXI TIANSHUN ECOLOGICAL AGRICULTURE CO) dilué dans 12 g de gomme xanthane à 2%. Procédé de coloration sur mèche de cheveu préalablement décolorée (mèche de référence):
La mèche est entièrement trempée dans cette solution visqueuse puis mise à 50 °C pendant 30 min. La mèche est ensuite rincée à l'eau chaude.

Résultat : nous n'observons pas d'effet colorant comparativement à la mèche de référence.

## Revendications

1. Utilisation d'une composition cosmétique pour colorer les cheveux en blond, ladite composition cosmétique comprenant un principe actif colorant choisi dans le groupe constitué par un extrait de Gardénia et une poudre de Gardénia, **caractérisée en ce que** :
a) l'extrait de Gardénia contient entre 1 et 10% en poids de crocines, préférentiellement entre 1 et 5% en poids, par rapport au poids de l'extrait sec ;
b) la poudre de Gardénia contient entre 1 et 10% en poids de crocines, préférentiellement entre 1 et 5%, par rapport au poids de la poudre ;
c) la composition présente un pH compris entre 4 et 8, et ;
d) la composition est dénuée de composé réducteur.

2. Utilisation selon la revendication 1, **caractérisée en ce que** la composition comprend un excipient cosmétiquement acceptable.

3. Utilisation selon l'une des revendications 1 à 2, **caractérisée en ce que** la composition est dénuée de composé alcalin fixateur.

4. Utilisation selon l'une des revendications 1 à 3, **caractérisé en ce que** l'extrait est un extrait de fruits de Gardénia.

5. Utilisation selon l'une des revendications 1 à 4, **caractérisée en ce que** l'extrait est un extrait aqueux ou alcoolique ou hydroalcoolique.

6. Utilisation selon l'une des revendications 1 à 5, **caractérisée en ce que** l'extrait est un extrait sec.

7. Utilisation selon l'une des revendications 1 à 3, **caractérisé en ce que** la poudre est une poudre de fruits de Gardénia, de préférence de fruits séchés de Gardénia.

8. Utilisation selon l'une des revendications 1 à 7 pour colorer en blond les cheveux préalablement décolorés.

9. Kit comprenant un extrait de Gardénia ou une poudre de Gardénia tels que définis dans une des revendications 1 à 7, un excipient cosmétiquement acceptable et une notice d'emploi pour une utilisation pour la coloration des cheveux en blond.

10. Méthode de traitement cosmétique destinée à colorer les fibres kératiniques en blond, consistant en l'administration d'une composition cosmétique telle que définie dans l'une des revendications 1 à 7.

11. Méthode de coloration des cheveux en blond, comprenant les étapes :
a) application d'une composition cosmétique telle que définie dans l'une des revendications 1 à 7 ;
b) temps de pause compris entre 15 min et 3 heures ;
c) rinçage à l'eau ;
d) éventuelle répétition des étapes a) à c) ;
e) éventuel séchage.

12. Méthode de coloration selon la revendication 11, **caractérisée en ce qu'**elle comprend une étape optionnelle, avant l'étape a) consistant en la préparation extemporanée de la composition par mélange des composants d'un kit tel que défini dans la revendication 9.

13. Utilisation selon l'une des revendications 1 à 7, **caractérisé en ce que** l'extrait est obtenu par un procédé de préparation comprenant les étapes suivantes :
- une extraction à partir de fruits de Gardénia, en particulier de fruits séchés, optionnellement en présence de pectinases, par un solvant choisi dans le groupe constitué par l'eau, l'éthanol et l'acétone, ainsi que leurs mélanges,
- une séparation solide-liquide,
- une éventuelle stérilisation du filtrat, et
- éventuellement évaporation du solvant à des températures inférieures à 80 °C.

14. Utilisation selon la revendication 13, **caractérisé en ce que** l'étape d'extraction est réalisée à un pH compris entre 4 et 8, de préférence, entre 5 et 7,5, avantageusement entre 5,5 et 7,5, typiquement à pH neutre.

## Patentansprüche

1. Verwendung einer kosmetischen Zusammensetzung zum Blondfärben der Haare, wobei die kosmetische Zusammensetzung ein färbendes Wirkstoffprinzip umfasst, das aus der Gruppe ausgewählt ist, die aus einem Gardenia-Extrakt und einem Gardenia-Pulver besteht, **dadurch gekennzeichnet, dass**:
a. der Gardenia-Extrakt zwischen 1 und 10 Gew.-%, vorzugsweise zwischen 1 und 5 Gew.-% Crocine in Bezug auf das Gewicht des Trockenextrakts enthält,
b. das Gardenia-Pulver zwischen 1 und 10 Gew.-%, vorzugsweise zwischen 1 und 5 % Crocine in Bezug auf das Gewicht des Pulvers enthält,
c. die Zusammensetzung einen pH aufweist, der zwischen 4 und 8 liegt und
d. die Zusammensetzung einer reduzierenden Verbindung entbehrt.

2. Verwendung nach Anspruch 1, **dadurch gekennzeichnet, dass** die Zusammensetzung einen kosmetisch akzeptablen Hilfsstoff umfasst.

3. Verwendung nach einem der Ansprüche 1 bis 2, **dadurch gekennzeichnet, dass** die Zusammensetzung einer alkalischen Fixierverbindung entbehrt.

4. Verwendung nach einem der Ansprüche 1 bis 3, **dadurch gekennzeichnet, dass** der Extrakt ein Extrakt aus Gardeniafrüchten ist.

5. Verwendung nach einem der Ansprüche 1 bis 4, **dadurch gekennzeichnet, dass** der Extrakt ein wässriger oder alkoholischer oder hydroalkoholischer Extrakt ist.

6. Verwendung nach einem der Ansprüche 1 bis 5, **dadurch gekennzeichnet, dass** der Extrakt ein Trockenextrakt ist.

7. Verwendung nach einem der Ansprüche 1 bis 3, **dadurch gekennzeichnet, dass** das Pulver ein Pulver aus Gardeniafrüchten, vorzugsweise aus getrockneten Gardeniafrüchten, ist.

8. Verwendung nach einem der Ansprüche 1 bis 7 zum Blondfärben der zuvor entfärbten Haare.

9. Set, umfassend einen Gardeniaextrakt oder ein Gardeniapulver, wie in einem der Ansprüche 1 bis 7 definiert, einen kosmetisch akzeptablen Hilfsstoff und eine Gebrauchsanleitung für eine Verwendung zum Blondfärben der Haare.

10. Kosmetische Behandlungsmethode, die zum Färben der Keratinfasern in Blond, bestimmt ist, die aus der Verabreichung einer kosmetischen Zusammensetzung besteht, wie in einem der Ansprüche 1 bis 7 definiert.

11. Methode zum Blondfärben der Haare, umfassend die Schritte:
a) Auftragen einer kosmetischen Zusammensetzung, wie in einem der Ansprüche 1 bis 7 definiert,
b) Einwirkzeit zwischen 15 Min. und 3 Stunden,
c) Ausspülen mit Wasser,
d) eventuelles Wiederholen der Schritte a) bis c),
e) eventuelles Trocknen.

12. Färbemethode nach Anspruch 11, **dadurch gekennzeichnet, dass** sie einen optionalen Schritt vor dem Schritt a) umfasst, der in der Herstellung der Zusammensetzung unmittelbar vor dem Gebrauch durch Mischen der Bestandteile eines wie in Anspruch 9 festgelegten Sets besteht.

13. Verwendung nach einem der Ansprüche 1 bis 7, **dadurch gekennzeichnet, dass** der Extrakt durch ein Herstellungsverfahren erhalten wird, das die folgenden Schritte umfasst:
- eine Extraktion aus Gardeniafrüchten, insbesondere getrockneten Früchten, optional bei Anwesenheit von Pektinasen, durch ein Lösungsmittel, das aus der Gruppe ausgewählt ist, die aus Wasser, Ethanol und Aceton sowie deren Gemischen besteht,
- eine Trennung Feststoff-Flüssigkeit,
- eine eventuelle Sterilisation des Filtrats, und
- eventuell Verdampfung des Lösungsmittels bei Temperaturen unter 80 °C.

14. Verwendung nach Anspruch 13, **dadurch gekennzeichnet, dass** der Extraktionsschritt bei einem pH, der zwischen 4 und 8, vorzugsweise zwischen 5 und 7,5, in vorteilhafter Weise zwischen 5,5 und 7,5 liegt, in typischer Weise bei neutralem pH durchgeführt wird.

## Claims

1. Use of a cosmetic composition for dyeing hair blonde, said cosmetic composition comprising a dye active principle selected from the group consisting of a Gardenia extract and a Gardenia powder, **characterized in that**:
a. the Gardenia extract contains between 1 and 10 wt% crocins, preferentially between 1 and 5 wt%, relative to the weight of the dry extract,
b. the Gardenia powder contains between 1 and 10 wt% crocins, preferentially between 1 and 5 wt%, relative to the weight of the powder,
c. the composition has a pH ranging between 4 and 8, and
d. the composition is free of reducing compound.

2. Use according to claim 1, **characterized in that** the composition comprises a cosmetically acceptable excipient.

3. Use according to one of claims 1 to 2, **characterized in that** the composition is free of alkaline fixing compound.

4. Use according to one of claims 1 to 3, **characterized in that** the extract is an extract of Gardenia fruits.

5. Use according to one of claims 1 to 4, **characterized in that** the extract is an aqueous or alcoholic or hydroalcoholic extract.

6. Use according to one of claims 1 to 5, **characterized in that** the extract is a dry extract.

7. Use according to one of claims 1 to 3, **characterized in that** the powder is a powder of Gardenia fruits, preferably of dried Gardenia fruits.

8. Use according to one of claims 1 to 7 for dyeing hair which has been bleached beforehand blonde.

9. A kit comprising a Gardenia extract or a Gardenia powder as defined in one of claims 1 to 7, a cosmetically acceptable excipient and a set of instructions for use for dyeing hair blonde.

10. A cosmetic treatment method for dyeing keratin fibers blonde consisting, in the administration of a cosmetic composition as defined in one of the claims 1 to 7.

11. A method for dyeing hair blonde, comprising the steps:
a) application of a cosmetic composition as defined in one of the claims 1 to 7,
b) waiting for a period of time ranging between 15 min and 3 hours,
c) rinsing with water,
d) optional repetition of steps a) to c),
e) optional drying.

12. The dyeing method according to claim 11, **characterized in that** it comprises an optional step, before step a), consisting in the preparation of the composition immediately before use by mixing the components of a kit as defined in claim 9.

13. Use according to one of claims 1 to 7, **characterized in that** the extract is obtained by a preparation process comprising the following steps:
- extraction from Gardenia fruits, in particular from dried fruits, optionally in the presence of pectinases, with a solvent selected from the group consisting of water, ethanol, acetone and mixtures thereof,
- solid-liquid separation,
- optional sterilization of the filtrate, and
- optional evaporation of the solvent at temperatures below 80°C.

14. Use according to claim 13, **characterized in that** the extraction step is performed at a pH ranging between 4 and 8, preferably between 5 and 7.5, advantageously between 5.5 and 7.5, typically at neutral pH.
